# EUROPEAN PATENT APPLICATION

(11) **EP 3 616 598 A1**
(43) Date of publication of application: **04.03.2020**
(21) Application number: 19194102.0
(22) Date of filing: 28.08.2019
(51) Int. Cl.: A61B 1/12, A61B 1/253, A61B 1/00, A61B 1/015

(54) **CAP AND IMAGING CAPTURING DEVICE**

(30) Priority: 31.08.2018 JP 2018162934
(71) Applicant: J. MORITA MANUFACTURING CORPORATION, Fushimi-ku, Kyoto-shi, Kyoto 6128533 (JP)
(72) Inventor: TANAKA, Tsuyoshi, Kyoto, 612-8533 (JP); WAKAZOME, Naonori, Kyoto, 612-8533 (JP)
(74) Representative: Müller Hoffmann & Partner

(57) **Abstract**

Provided is an image capturing device (100) for capturing an image of a target (99). The image capturing device (100) includes: a tubular portion (20) including an optical component (2) and configured to receive light from the target (99); a housing (30) including an electronic component (40) configured to process the light received from the tubular portion (20); a cap (10) covering at least a part of the tubular portion (20); and a blower (37) mounted in the housing (30) and configured to supply air to at least one of the cap (10) and the optical component (2) included in the tubular portion (20). The cap (10) and the part of the tubular portion (20) covered by the cap (10) define an air-feeding path (11) configured to pass the air supplied from the blower (37).

## Description

### BACKGROUND OF THE INVENTION

### Field of the Invention

The present invention relates to a cap for covering a part of an image capturing device and to an image capturing device.

### Description of the Background Art

In the field of dentistry, a variety of devices have been and are being developed as image capturing devices for capturing an image of the inside of an oral cavity. For example, in order to digitally design a prosthesis or the like on a computer, an image capturing device has been developed for acquiring a three-dimensional shape of a tooth/teeth. This image capturing device is disclosed for example in US Patent No. 7,255,558 and called three-dimensional scanner or intraoral scanner.

A tip of the image capturing device may be equipped with a probe to be inserted in an oral cavity for capturing an image of the inside of the oral cavity. This probe includes an optical component and is made up of, for example, a tubular portion for receiving light from a target to be imaged, and a cap covering the tubular portion.

This probe is inserted in an oral cavity for capturing an image of the inside of the oral cavity. Due to high temperature and high humidity in the oral cavity, the optical component (such as mirror, for example) included in the probe is fogged. With the fogged optical component of the probe, the image capturing device cannot successfully capture an image of the inside of the oral cavity.

### SUMMARY OF THE INVENTION

US Patent No. 7,255,558 discloses an image capturing device having air-feeding tubes disposed on respective sides of a probe for feeding air from the air-feeding tubes. For the image capturing device, it is not an object of feeding air to prevent fogging of an optical component of the probe. The image capturing device disclosed in US Patent No. 7,255,558 is structured to have, on respective sides of the probe, the air-feeding tubes for feeding air. Such an image capturing device cannot be manufactured easily by molding or the like, and the quality is difficult to improve. For the image capturing device disclosed in US Patent No. 7,255,558, a cap having a complicated structure may not be sterilized sufficiently in a sterilization process, even when the cap is detachable from the image capturing device.

The present invention has been made to solve the above-described problem. An object of the invention is to prevent fogging of an optical component mounted in a tip of an image capturing device, and to provide a cap having an easy-to-manufacture structure as well as an image capturing device.

According to the present invention, an image capturing device for capturing an image of a target includes a tubular body, a housing, a cap, and a blower. The tubular portion includes an optical component and is configured to receive light from the target. The housing includes an electronic component and is configured to process the light received from the tubular portion. The cap covers at least a part of the tubular portion. The blower is mounted in the housing and is configured to supply air to at least one of the cap and the optical component included in the tubular portion. The cap and the part of the tubular portion covered by the cap define an air-feeding path configured to pass the air supplied from the blower.

According to the present invention, a cap is mountable on and detachable from a tubular portion of an image capturing device. The image capturing device includes: the tubular portion including an optical component and configured to receive light from a target; a housing including an electronic component configured to process the light received from the tubular portion; and a blower mounted in the housing and configured to supply air. The cap includes: a reflector configured to direct the light from the target to the tubular portion; and a cover member holding the reflector at one end while covering the tubular portion. A groove is formed in at least a part of an inner surface, facing the tubular portion, of the cover member. With the cap mounted on the tubular portion, the groove forms an air-feeding path configured to supply the air from the blower to at least one of the reflector and the optical component included in the tubular portion.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic diagram for illustrating a configuration of a three-dimensional scanner according to a first embodiment.
Fig. 2 is a block diagram showing the configuration of the three-dimensional scanner according to the first embodiment.
Fig. 3 is a schematic diagram for illustrating a configuration of an air-feeding path formed in a probe according to the first embodiment.
Fig. 4 is a cross-sectional view for illustrating flow of air in a tip of a cap according to the first embodiment.
Fig. 5 is a schematic diagram for illustrating the size of an air-feeding path formed in the probe according to the first embodiment.
Figs. 6A to 6D are each a cross-sectional view for illustrating the size of the air-feeding path formed in the probe according to the first embodiment.
Fig. 7 is a schematic diagram for illustrating flow of air in an air-feeding path formed in the probe according to the first embodiment.
Fig. 8 is a cross-sectional view for illustrating flow of air in the case where a flow regulator plate is mounted in a tip of a cap according to a second embodiment.
Figs. 9A and 9B are each a schematic diagram for illustrating a configuration of a plurality of air-feeding paths formed in a probe according to a third embodiment.
Fig. 10 is a schematic diagram for illustrating a configuration of an air-feeding path formed in a probe according to a fourth embodiment.

### DESCRIPTION OF THE PREFERRED EMBODIMENTS

Embodiments of the present disclosure are hereinafter described in detail with reference to the drawings. In the drawings, the same or corresponding parts are denoted by the same reference characters, and a description thereof is not repeated.

### First Embodiment

An image capturing device according to a first embodiment is a three-dimensional scanner (intraoral scanner) for acquiring a three-dimensional shape of a tooth/teeth in an oral cavity. The image capturing device according to the present invention, however, is not limited to the three-dimensional scanner for an oral cavity, but is applicable to any image capturing device having a similar configuration. For example, the image capturing device of the present invention is also applicable to a three-dimensional scanner capable of capturing an image of the inside of a human ear besides the inside of an oral cavity for acquiring a three-dimensional shape of the inside of the ear.

### [Configuration of Three-Dimensional Scanner]

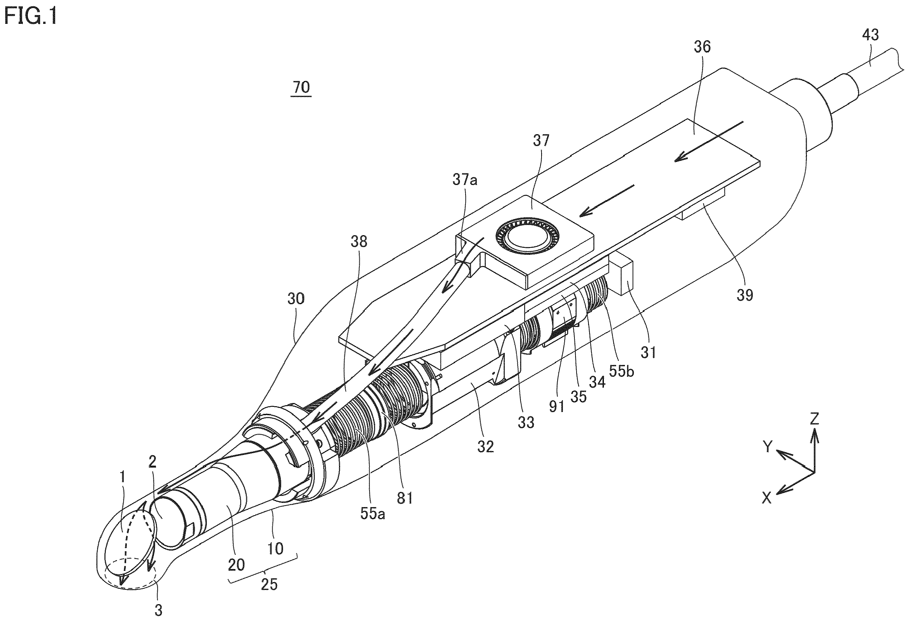

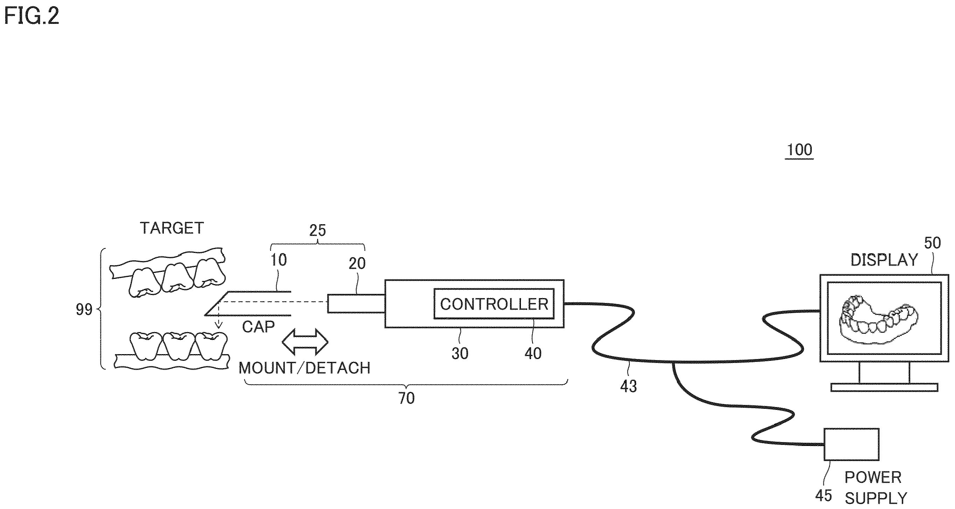

Fig. 1 is a schematic diagram for illustrating a configuration of a three-dimensional scanner 100 according to the first embodiment. Fig. 2 is a block diagram showing the configuration of three-dimensional scanner 100 according to the first embodiment. As shown in Fig. 2, three-dimensional scanner 100 includes a cap 10, a tubular portion 20, a housing 30, a controller 40, a power supply 45, and a display 50. Power supply 45 and display 50 are connected by a cable 43 to a hand piece 70 that includes cap 10, tubular portion 20, housing 30, and controller 40. The sole configuration of hand piece 70 may also be called three-dimensional scanner.

Cap 10 is inserted in an oral cavity in which a target 99 to be imaged is located. Then, cap 10 projects light having a pattern (this light is also referred to "pattern" hereinafter), and directs the reflected light from target 99 on which the pattern is projected, to tubular portion 20 and housing 30. Moreover, cap 10 is mountable on and detachable from tubular portion 20, and therefore, only cap 10 which may be brought into contact with a living body can be detached from tubular portion 20 to be subjected to sterilization (treated in a high-temperature high-humidity environment, for example) for preventing infection. Cap 10 is made up of a mirror 1 and a cover member that holds mirror 1 at one end while covering tubular portion 20.

Tubular portion 20 has a shape that can be fit in cap 10. Tubular portion 20 is a portion protruding from housing 30. Tubular portion 20 includes an optical component(s) such as relay lens and λ/4 plate for directing light received by cap 10 to housing 30. Cap 10 and tubular portion 20 constitute a probe 25. Tubular portion 20 may be a part of housing 30 or a part separate from housing 30.

Housing 30 projects a pattern on target 99 through cap 10 and captures an image of the projected pattern. Housing 30 includes an optical component and a light source for generating a pattern to be projected on target 99, a lens component for focusing the pattern on the surface of target 99, a focus adjustment mechanism for adjusting the focus of the lens, and an image capturing device for capturing an image of the projected pattern, for example. Housing 30 is described as being configured to acquire a three-dimensional shape by the focusing technique. Housing 30, however, is not limited to this configuration but may be configured to acquire a three-dimensional shape by a technique such as confocal method, triangulation, white light interferometry, stereo method, photogrammetry, SLAM (Simultaneous Localization and Mapping), or optical coherence tomography (OCT). In other words, housing 30 configured on the basis of any principle is applicable as long as it is configured to acquire a three-dimensional shape by an optical technique.

Controller 40 is contained in housing 30, controls respective operations of mechanical parts and electronic parts disposed in housing 30, and processes the image captured by housing 30 to acquire a three-dimensional shape. Controller 40 includes a CPU (Central Processing Unit) as a control center, a ROM (Read Only Memory) storing programs and control data for example for the CPU to operate, a RAM (Random Access Memory) serving as a work area for the CPU, a GPU (Graphics Processing Unit) chiefly performing image processing, and an input/output interface for ensuring signal consistency with peripherals, for example. The CPU and/or the GPU may be configured in the form of an FPGA (Field Programmable Gate Array). Controller 40 is also able to output the acquired three-dimensional shape to display 50, and information such as settings for housing 30 can be input to controller 40 by an input device or the like (not shown).

At least a part of arithmetic operations for acquiring the three-dimensional shape by processing the captured image may be implemented as software by the CPU of controller 40, or implemented as hardware that performs processing separately from the CPU. At least a part of the processor such as CPU or hardware may be incorporated in housing 30. While Fig. 2 depicts each component (30, 50, 45) of three-dimensional scanner 100 as being connected by cable 43, a part or the whole of the connection may be implemented by radio communication. Controller 40 can be a separate component rather than being contained in housing 30 to thereby reduce the weight of hand piece 70.

Power supply 45 is a device for supplying electric power to drive housing 30 and controller 40. Power supply 45 may be mounted outside controller 40 as shown in Fig. 1, or mounted inside controller 40. Power supply 45 may include a plurality of power supplies so that electric power can be fed separately to housing 30 and display 50.

Display 50 is a display device for showing the resultant three-dimensional shape of target 99 obtained by controller 40. Display 50 can also be used as a display device for showing other kinds of information such as information about settings for housing 30, information about a patient, activation state of the scanner, manual, help screen, for example. As display 50, a stationary liquid crystal display, a head-mount display, or a glasses-type wearable device, for example, is applicable. Display 50 may be a plurality of displays, and the results of measurement of the three-dimensional shape and/or other kinds of information may be displayed simultaneously or in the divided form on a plurality of displays 50.

### [Configuration of Hand Piece]

Next, a configuration of hand piece 70 is described in detail. In Fig. 1, the longitudinal direction of hand piece 70 is X axis, the width direction of hand piece 70 is Y axis, and the height of hand piece 70 is Z axis. The X-axis direction is parallel to the optical axis of lens 81 described later herein.

As shown in Fig. 1, hand piece 70 is made up of probe 25 and housing 30, and connected by cable 43 to power supply 45 and display 50. Housing 30 has an air inlet (not shown) formed in its surface connected with cable 43, and the outside air is thus allowed to enter housing 30. The air inlet may at least allow the outside air to enter housing 30, and the shape and the number of air inlet(s) are not particularly limited.

In hand piece 70, housing 30 in the shape of a column is of a type (pen type) to be used by a user holding a part of housing 30 from above (upper side as seen in the drawings) or from below (lower side as seen in the drawings). The user holds housing 30 itself to insert the tip of probe 25 into an oral cavity and measure the shape of target 99. As to the width of hand piece 70, the lower part may be narrower than the upper part in consideration of ease of holding and ease of operation by users.

In hand piece 70, a handle may be mounted on a part of housing 30. For hand piece 70 having a handle, a user may grip the handle to insert the tip of probe 25 into an oral cavity to measure the shape of target 99.

Probe 25 is made up of cap 10 and tubular portion 20. Cap 10 is made up of mirror 1 and a cover member that holds mirror 1 at one end while covering tubular portion 20. At the tip of cap 10 where mirror 1 is located, a light inlet 3 is formed. Light inlet 3 is a window for projecting light from light source 31 on target 99 and for allowing reflected light from target 99 to enter the cap. Mirror 1 reflects light from light source 31 toward target 99 and also reflects light allowed to enter from light inlet 3 toward housing 30.

Housing 30 contains a large number of electronic components such as CPU 34 as controller 40, an image sensor 33, a GPU (not shown), a power supply circuit 39, and other electronic components (not shown). Among the electronic components, CPU 34, image sensor 33, the GPU (not shown), and power supply circuit 39 generate a greater amount of heat than other electronic components. Among these electronic components, particularly CPU 34 and the GPU (not shown) are operational electronic components that generate a greater amount of heat. CPU 34 is therefore equipped with a heat sink 35 for releasing heat. These electronic components are mounted on an electronic circuit board 36.

Housing 30 contains a blower 37 and an air-feeding tube 38. Blower 37 is mounted so that blower 37 is located higher than the electronic components when a user holds housing 30 for using three-dimensional scanner 100. For example, in Fig. 1, blower 37 is located higher, as seen in the drawing, than electronic components such as CPU 34, image sensor 33, the GPU (not shown), and power supply circuit 39. Air allowed to enter from the air inlet is heated by the heat generated from the electronic components to stay in an upper part in housing 30. The heated air is introduced into blower 37 as indicated by the arrows in Fig. 1, passed through air-feeding tube 38 connected to an air outlet 37a formed in blower 37 to be fed to the tip of probe 25. In probe 25, the air is passed through an air-feeding path defined by cap 10 and a part of tubular portion 20 covered by cap 10.

Probe 25 inserted in an oral cavity may not accurately measure the shape of target 99 due to high temperature and high humidity in the oral cavity that causes fogging of optical components (such as mirror 1 and a phase retardation plate 2 (quarter wavelength plate) mounted in tubular portion 20, for example) located in the tip of probe 25. In contrast, three-dimensional scanner 100 according to the first embodiment feeds air to the tip of probe 25 by blower 37 to prevent fogging of the optical components mounted in cap 10 and tubular portion 20. Particularly hot air can be fed to the tip of probe 25 to decrease the difference between the temperature in the oral cavity and the surface temperature of the optical components mounted in cap 10 and tubular portion 20 to thereby prevent fogging more effectively.

Housing 30 contains, as an image capturing unit, a light source 31, a prism 32, lens 81, and image sensor 33. The light that is output from light source 31 passes through prism 32 and lens 81 to be projected by mirror 1 toward target 99. The light reflected from target 99 is allowed by light inlet 3 to enter probe 25, and is directed by mirror 1 to tubular portion 20. The light directed to tubular portion 20 passes through phase retardation plate 2, lens 81, and prism 32 to be detected by image sensor 33. When a three-dimensional shape is to be acquired by the focusing technique, the light passed through a pattern generation element (not shown) located between lens 81 and target 99 is projected on target 99. Reciprocation of lens 81 in the X-axis direction causes change of the focus point of the projected pattern. For each change of the focus position, image sensor 33 detects the light from target 99. Based on the position of lens 81 and the result of detection by image sensor 33 at the time, controller 40 calculates the shape information of target 99.

As lens 81 fixed by a spring 55a reciprocates in the X-axis direction, the barycenter position of hand piece 70 is moved by the mass of lens 81, which is conveyed in the form of vibration to the user holding this hand piece 70. In order to cancel this vibration, a counterweight 91 is further mounted in housing 30. Counterweight 91 is secured by a spring 55b, and placed in the direction in which lens 81 is moved linearly so as not to interfere with the optical path between target 99 and lens 81 and the optical path between lens 81 and image sensor 33.

Blower 37 is a blower fan having a small pressure loss and suitable for feeding air into a narrow duct. The blower fan is capable of introducing air in the direction of the rotational axis and feeding the air in the direction orthogonal to the rotational axis. Blower 37 may be any as long as it has a small pressure loss and is suitable for feeding air into a narrow duct. For example, blower 37 may be diaphragm pump, PZT micro blower, or the like, as long as it is suitable for feeding air into a narrow duct.

### [Configuration of Air-Feeding Path]

As described above, when an image of the inside of an oral cavity is to be captured by three-dimensional scanner 100, probe 25 is inserted in the oral cavity. When probe 25 is inserted in the oral cavity, the optical component in tubular portion 20 and mirror 1 mounted in cap 10, for example, are fogged, due to high temperature and high humidity in the oral cavity. The fogging of the optical component in tubular portion 20 and mirror 1 mounted in cap 10 for example results in failure to successfully capture an image of a pattern projected on target 99. As a result, the three-dimensional shape of target 99 cannot be acquired with high accuracy.

In view of the above, three-dimensional scanner 100 according to the present embodiment prevents fogging by supplying, to the optical component in tubular portion 20 and mirror 1 mounted in cap 10, for example, the air fed from blower 37 mounted in housing 30. Specifically, the air fed from blower 37 passes through air outlet 37a and air-feeding tube 38 to reach probe 25. The air reaching probe 25 then passes through an air-feeding path formed in probe 25 to be supplied to the optical component in tubular portion 20 and mirror 1 mounted in cap 10, for example. Air-feeding tube 38 may be a soft tube (silicon, urethane, polytetrafluoroethylene, or the like) or a hard tube (stainless, aluminum, copper, or the like).

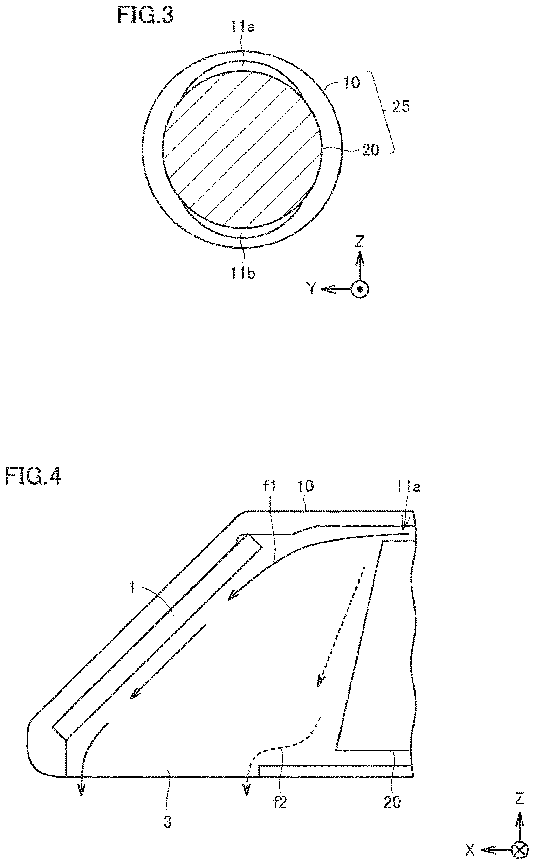

The air-feeding path formed in probe 25 is defined by cap 10 and a part of tubular portion 20 that is covered by cap 10. Fig. 3 is a schematic diagram for illustrating the configuration of the air-feeding path formed in probe 25 according to the first embodiment. In the schematic diagram shown in Fig. 3, a Y-Z cross section of probe 25 is shown. An essential function of the cap is to cover the tubular portion and there should essentially be almost no gap between the cap and the tubular portion. In contrast, in cap 10 according to the first embodiment, grooves 11a, 11b forming an air-feeding path are formed in at least a part of the inner surface of cap 10 that faces tubular portion 20 when cap 10 is mounted on tubular portion 20. In other words, grooves 11a, 11b are formed in the cover member forming cap 10.

In probe 25, an air-feeding path for allowing air to flow is formed by grooves 11a, 11b formed in cap 10. Therefore, the air fed from blower 37 passes through the air-feeding path formed in probe 25 to be supplied to the optical component in tubular portion 20 and mirror 1 mounted in cap 10, for example. Three-dimensional scanner 100 prevents fogging of the optical component in tubular portion 20 and mirror 1 mounted in cap 10, for example, by supplying air to the optical component in tubular portion 20 and mirror 1 mounted in cap 10, for example.

Fig. 4 is a cross-sectional view for illustrating a flow of air in the tip of cap 10 according to the first embodiment. In Fig. 4, an X-Z cross section of a part of cap 10 and tubular portion 20 is shown. The air sent from the air-feeding path formed by upper groove 11a in Fig. 4 includes a flow f1 supplied to the surface of mirror 1 mounted in cap 10 to thereafter reach light inlet 3, and a flow f2 supplied to the surface of an optical component (λ/4 plate, for example) mounted in the tip of tubular portion 20 to thereafter reach light inlet 3. In other words, flow f1 of air is used to prevent fogging of mirror 1 mounted in cap 10 and flow 2 of air is used to prevent fogging of the optical component in tubular portion 20. Further, air is also fed from light inlet 3 of probe 25 to a tooth/teeth as target 99, and therefore, the air can also be used to dry moisture on the surface of the tooth/teeth. Moisture on the surface of the tooth/teeth can be dried to increase reflection of light from the surface of the tooth/teeth to thereby enable improvement of the measurement precision of three-dimensional scanner 100.

Further, air that is sent from light inlet 3 of probe 25 also produces the effect of preventing saliva for example in the oral cavity from entering hand piece 70. Therefore, the air sent from light inlet 3 of probe 25 also functions to prevent infection. Moreover, blower 37 discharges air from inside hand piece 70 to the outside to cool the heat generated from the electronic components in housing 30 and thus produce the effect of decreasing the temperature in housing 30 (cooling effect). Blower 37 enhances the effect of preventing fogging by supplying, to the optical component in tubular portion 20 and mirror 1 mounted in cap 10, for example, the air heated in housing 30.

The air-feeding path formed in probe 25 is defined between grooves 11a, 11b formed in cap 10 and tubular portion 20, and therefore, it is unnecessary to add a structure such as a tube that passes air, for example, to cap 10. Cap 10 can thus have an easy-to-manufacture structure. The easy-to-manufacture structure of cap 10 enables cap 10 to be manufactured by molding, for example, and enables improvement of the quality and sufficient sterilization in a sterilization process.

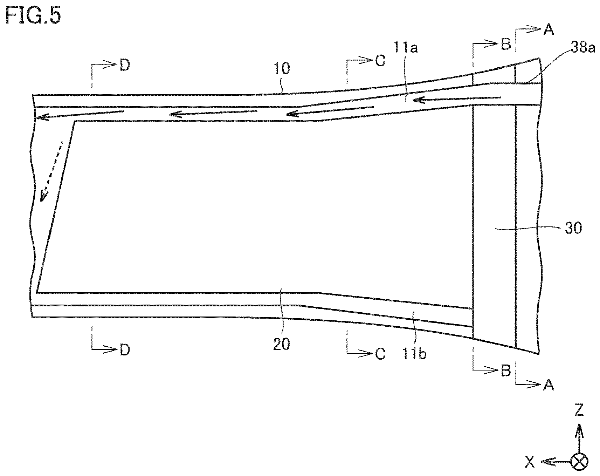

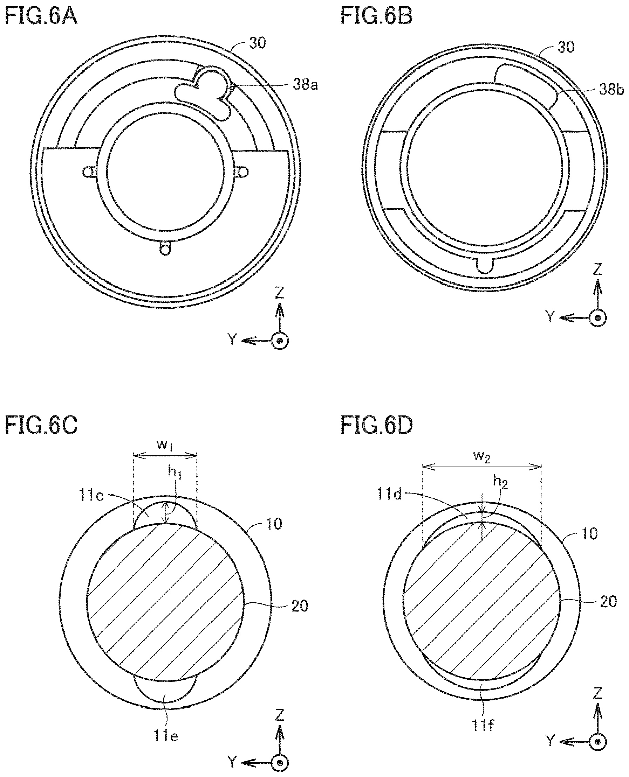

In order to efficiently supply air fed from blower 37 to the optical component in tubular portion 20 and mirror 1 mounted in cap 10, for example, the air-feeding path formed in probe 25 has a substantially constant cross section facing the air-feeding direction (X-axis direction). Fig. 5 is a schematic diagram for illustrating the size of the air-feeding path formed in probe 25 according to the first embodiment. Figs. 6A to 6D are each a cross-sectional view for illustrating the size of the air-feeding path formed in probe 25 according to the first embodiment. In Fig. 5, an X-Z cross section of a part of housing 30 and a part of probe 25 is shown. In Figs. 6A to 6D, an A cross section, a B cross section, a C cross section, and a D cross section along an A-A line, a B-B line, a C-C line, and a D-D line indicated in Fig. 5 are shown, respectively, as seen in the direction of the arrows in Fig. 5.

The A cross section shown in Fig. 6A is a cross section of housing 30 in the vicinity of the boundary with probe 25. Housing 30 in this cross section has a connection port 38a for connecting with air-feeding tube 38. The B cross section shown in Fig. 6B is a cross section of housing 30 that is closer to the boundary with probe 25 than the A cross section is. Housing 30 in this cross section has an air outlet 38b communicating with connection port 38a for discharging the air into the air-feeding path of probe 25.

The C cross section shown in Fig. 6C is a cross section of probe 25 relatively closer to housing 30. Probe 25 in this section has grooves 11c, 11e formed in cap 10. Between grooves 11c, 11e formed in cap 10 and tubular portion 20, the air-feeding path of probe 25 is defined. Grooves 11c, 11e in the C cross section have width w₁ and depth h₁. In other words, the air-feeding path of probe 25 in the C cross section has width w₁ and height h₁. The height of the air-feeding path is herein the length measured from tubular portion 20 to cap 10. The width of the air-feeding path is herein the length in the direction perpendicular to the height measured from tubular portion 20 to cap 10.

The D cross section shown in Fig. 6D is a cross section of probe 25 relatively further from housing 30. Probe 25 in this cross section has grooves 11d, 11f formed in cap 10. Between grooves 11d, 11f formed in cap 10 and tubular portion 20, the air-feeding path of probe 25 is defined. Grooves 11d, 11f in the D cross section has width w₂ and depth h₂. In other words, the air-feeding path of probe 25 in the D cross section have width w₂ and height h₂.

It is seen, from a comparison between the size of the air-feeding path of probe 25 in the C cross section and the size of the air-feeding path of probe 25 in the D cross section, the width meets w₁ < w₂ and the height meets h₁ > h₂. Specifically, in the direction from housing 30 to cap 10, the width of the air-feeding path of probe 25 gradually increase and the height thereof gradually decreases. It should be noted that the cross-sectional area of the air-feeding path in the C cross section is substantially identical to the cross-sectional area of the air-feeding path in the D cross section. The areas that are substantially identical to each other herein include the areas completely identical to each other and also the areas having a difference therebetween within an error (within 1 cm² for example) due to manufacturing, for example.

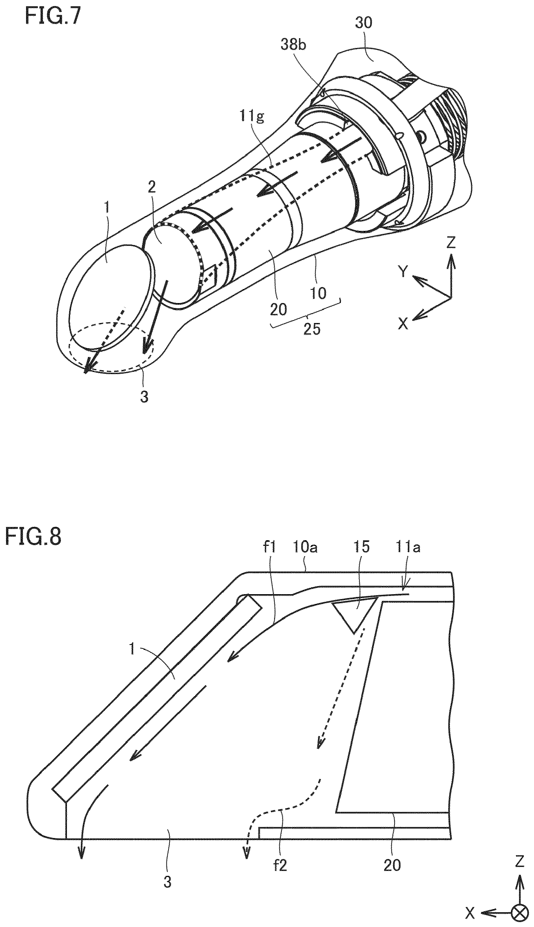

Fig. 7 is a schematic diagram for illustrating a flow of air in the air-feeding path formed in probe 25 according to the first embodiment. The air-feeding path of probe 25 is formed to gradually increase in width and gradually decrease in height in the direction from housing to cap 10, to thereby have the shape of an air-feeding path 11g as shown in Fig. 7. Air feeding path 11g has a shape (open-fan-like shape, for example) expanding the air discharged from air outlet 38b to have its width, in the vicinity of an optical component mounted in the tip of tubular portion 20, substantially identical to or larger than the width of this optical component, or air-feeding path 11g has a shape expanding the air discharged from air outlet 38b to have its width, in the vicinity of mirror 1 mounted in cap 10, substantially identical to or larger than the width of mirror 1. Thus, the air passing through air-feeding path 11g of probe 25 is supplied to the whole of the optical component mounted in the tip of tubular portion 20 and mirror 1 mounted in cap 10. Therefore, three-dimensional scanner 100 can prevent fogging of the whole of the optical component and mirror 1 or fogging of a greater extent. Here, the widths that are substantially identical to each other herein include the widths completely identical to each other and also the widths having a difference therebetween within an error (within 5 mm for example) due to manufacturing, for example.

As seen from the foregoing, three-dimensional scanner 100 according to the first embodiment includes: tubular portion 20 including an optical component and configured to receive light from a target; housing 30 including an electronic component configured to process the light received from tubular portion 20; cap 10 covering at least a part of tubular portion 20; and blower 37 mounted in housing 30 and configured to supply air to at least one of cap 10 and the optical component included in tubular portion 20. Cap 10 and the part of tubular portion 20 covered by cap 10 define an air-feeding path configured to pass the air supplied from blower 37. Accordingly, three-dimensional scanner 100 according to the first embodiment can prevent fogging of the optical component mounted in the tip (cap 10 and the optical component included in tubular portion 20), and can provide cap 10 having an easy-to-manufacture structure.

Cap 10 is mountable on and detachable from tubular portion 20 and can therefore be sterilized. Three-dimensional scanner 100 according to the first embodiment is not limited to the three-dimensional scanner with cap 10 mountable on and detachable from tubular portion 20, but may be a three-dimensional scanner with cap 10 fixed relative to tubular portion 20, or with tubular portion 20 and cap 10 integrated into a single probe 25.

Cap 10 mountable on and detachable from tubular portion 20 of three-dimensional scanner 100 includes mirror 1 configured to direct light from the target to tubular portion 20, and a cover member holding mirror 1 at one end while covering tubular portion 20. Grooves 11a, 11b are formed in at least a part of the inner surface, facing tubular portion 20, of the cover member. With cap 10 mounted on tubular portion 20, grooves 11a, 11b form an air-feeding path configured to supply the air from blower 37 to at least one of mirror 1 and the optical component included in tubular portion 20. Accordingly, while cap 10 in the first embodiment has an easy-to-manufacture structure, cap 10 mounted on tubular portion 20 enables prevention of fogging of the optical component mounted in the tip (cap 10 and optical component included in tubular portion 20).

The air-feeding path has a substantially constant cross-sectional area facing the air-feeding direction in the air-feeding path. Accordingly, the air-feeding path is capable of efficiently supplying the air fed from blower 37 to the optical component of tubular portion 20 and mirror 1 mounted in cap 10, for example. The shape of the air-feeding path is not limited to the shape having a substantially constant cross-sectional area facing the air-feeding direction in the air-feeding path, but may be another shape.

Further, the air-feeding path may be shaped to have a height measured from tubular portion 20 to cap 10 (height: depth of the groove), and the height may decrease gradually in the direction from housing 30 to cap 10. Moreover, in the vicinity of the optical component included in tubular portion 20, the width of the air-feeding path may be substantially identical to or more than the width of the optical component included in tubular portion 20. Further, in the vicinity of mirror 1 of cap 10, the width of the air-feeding path may be substantially identical to or more than the width of mirror 1.

According to the foregoing description, three-dimensional scanner 100 in the first embodiment is configured to capture an image of the inside of an oral cavity, and an optical component of cap 10 is mirror 1 configured to reflect light from a target (tooth/teeth, for example) in the oral cavity toward tubular portion 20. Three-dimensional scanner 100 in the first embodiment is not limited to this but may be a three-dimensional scanner applied to any use other than intraoral imaging.

### Second Embodiment

According to the above description of cap 10 in the first embodiment, the air that is sent from the air-feeding path formed by groove 11a includes a flow to the surface of mirror 1 and a flow to the optical component mounted in the tip of tubular portion 20 as shown in Fig. 4. Regarding a second embodiment, a cap is described that branches the air sent from the air-feeding path into flows as intended. Fig. 8 is a cross-sectional view for illustrating flow of air in the case where a flow regulator plate 15 is placed in the tip of a cap 10a according to the second embodiment. In cap 10a in the second embodiment, the same part as that in cap 10 in the first embodiment shown in Fig. 4 is denoted by the same reference character, and the detailed description thereof is not repeated.

In cap 10a shown in Fig. 8, a flow regulator plate 15 is placed in the vicinity of the exit of the air-feeding path formed by groove 11a on the upper side as seen in the drawing, for regulating a flow of air. Flow regulator plate 15 is mounted to extend in the Y-axis direction and the shape of its X-Z cross section is a triangular shape. Flow regulator plate 15 is configured to branch the air that is sent from the air-feeding path formed by groove 11a into air flowing between flow regulator plate 15 and cap 10a and air flowing between flow regulator plate 15 and an optical component mounted in the tip of tubular portion 20. Specifically, the air flowing between flow regulator plate 15 and cap 10a forms the air of flow f1 supplied to a surface of mirror 1 in cap 10a to thereafter reach light inlet 3. In contrast, the air flowing between flow regulator plate 15 and the optical component mounted in the tip of tubular portion 20 forms the air of flow f2 supplied to a surface of the optical component in the tip of tubular portion 20 to thereafter reach light inlet 3.

Cap 10a is equipped with flow regulator plate 15 to branch the air sent from the air-feeding path into two flows as intended, to thereby prevent fogging of mirror 1 mounted in cap 10a by the air of flow f1 and prevent fogging of the optical component of tubular portion 20 by the air of flow f2. In cap 10a, the amount of air of flow f1 and the amount of air of flow f2 can be increased as compared with cap 10 without flow regulator plate 15.

As seen from the foregoing, cap 10a in the second embodiment includes flow regulator plate 15 (regulator) for regulating the air supplied through the air-feeding path to cause the air to flow to at least one of cap 10a and the optical component mounted in tubular portion 20. Therefore, with cap 10a in the second embodiment, the amount of air supplied to cap 10a and the optical component mounted in tubular portion 20 can be increased as compared with cap 10 without flow regulator plate 15. While flow regulator plate 15 shown in Fig. 8 is configured to branch the air sent from the air-feeding path into the air flowing between flow regulator plate 15 and cap 10a and the air flowing between flow regulator plate 15 and the optical component mounted in the tip of tubular portion 20, flow regulator plate 15 may also be configured to cause the air to flow to any one of them, without branching the air. As to the shape of flow regulator plate 15, its cross-sectional shape is not limited to the triangular shape, but may be any shape as long as it can regulate the flow of air.

### Third Embodiment

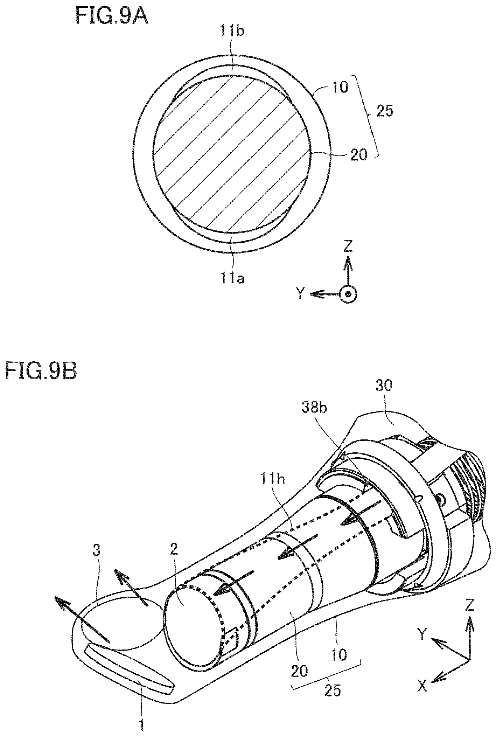

According to the description of cap 10 in the first embodiment, the air fed from blower 37 mounted in housing 30 is supplied through the air-feeding path formed by groove 11a shown in Fig. 3 to the optical component of tubular portion 20 and mirror 1 mounted in cap 10, for example. In connection with a third embodiment, a case is described where a plurality of grooves are formed in the cap to form a plurality of air-feeding paths between the grooves and the tubular portion. Figs. 9A and 9B are each a schematic diagram for illustrating a configuration of a plurality of air-feeding paths formed in probe 25 according to the third embodiment. In probe 25 in the third embodiment, the same part as that in probe 25 in the first embodiment shown in Figs. 3 and 7 is denoted by the same reference character, and the detailed description thereof is not repeated.

As shown in Fig. 9B, cap 10 is mounted on tubular portion 20 so that light inlet 3 of cap 10 faces upward as seen in the drawing. In other words, cap 10 is mounted on tubular portion 20 in an inverted manner relative to cap 10 shown in Fig. 7 with its light inlet 3 facing downward as seen in the drawing. Therefore, in a Y-Z cross section of probe 25 shown in Fig. 9A, groove 11b formed in the cover member which forms cap 10 is located on the upper side as seen in the drawing.

Accordingly, an air-feeding path 11h defined by groove 11b and tubular portion 20 is connected to air outlet 38b of housing 30 to allow the air fed from blower 37 to be supplied to the optical component of tubular portion 20 and mirror 1 mounted in cap 10, for example. Air-feeding path 11h has the shape as shown in Fig. 9B. Air-feeding path 11h has a shape expanding the air discharged from air outlet 38b to have its width, in the vicinity of the optical component mounted in the tip of tubular portion 20, substantially identical to or larger than the width of the optical component, or air-feeding path 11h has a shape expanding the air discharged from air outlet 38b to have its width, in the vicinity of mirror 1 mounted in cap 10, substantially identical to or larger than the width of mirror 1. Thus, the air passing through air-feeding path 1h of probe 25 is supplied to the whole of the optical component mounted in the tip of tubular portion 20 and mirror 1 mounted in cap 10. Therefore, three-dimensional scanner 100 can prevent fogging of the whole of the optical component and mirror 1 or fogging of a greater extent. Here, the widths that are substantially identical to each other herein include the widths completely identical to each other and also the widths having a difference therebetween within an error (within 5 mm for example) due to manufacturing, for example.

As seen from the foregoing, a plurality of grooves 11a, 11b are formed in at least a part of the inner surface, facing tubular portion 20, of cap 10 mounted on tubular portion 20, for forming a plurality of air-feeding paths. In other words, in the cover member that forms cap 10, a plurality of grooves 11a, 11b are formed. As shown in Figs. 9A and 9B, with a plurality of grooves formed in cap 10, the air-feeding paths for supplying the air fed from blower 37 to the optical component of tubular portion 20 and mirror 1 mounted in cap 10, for example, can be ensured even when the orientation of cap 10 mounted on tubular portion 20 is changed.

The number of grooves formed in cap 10 is not limited to two as shown in Fig. 9A but may be three or more. As long as the orientation of cap 10 mounted on tubular portion 20 will not be changed, a single groove may be formed in cap 10. When a plurality of grooves are formed in cap 10, these grooves may differ from each other in terms of the size and/or the shape of the groove.

Thus, a plurality of air-feeding paths are formed between cap 10 and tubular portion 20 according to the third embodiment. Therefore, even when the orientation of cap 10 mounted on tubular portion 20 is changed, the air fed from blower 37 can be supplied to the optical component of tubular portion 20 and mirror 1 mounted in cap 10, for example, through any of the air-feeding paths.

### Fourth Embodiment

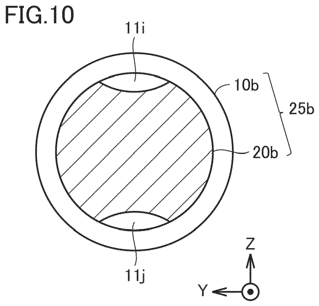

According to the above description of cap 10 in the first embodiment, the air fed from blower 37 mounted in housing 30 is supplied to the optical component of tubular portion 20 and mirror 1 mounted in cap 10, for example, through the air-feeding path formed by groove 11a shown in Fig. 3. In connection with a fourth embodiment, a configuration is described in which the air fed from the blower is supplied to the optical component of the tubular portion and the mirror mounted in the cap, for example, through an air-feeding path formed by a groove formed in the tubular portion. Fig. 10 is a schematic diagram for illustrating a configuration of an air-feeding path formed in a probe 25b according to the fourth embodiment. In probe 25b in the fourth embodiment, the same part as that in probe 25 in the first embodiment shown in Fig. 3 is denoted by the same reference character, and the detailed description thereof is not repeated.

In the schematic diagram shown in Fig. 10, a Y-Z cross section of probe 25b is shown. In cap 10 shown in Fig. 3 mounted on tubular portion 20, grooves 11a, 11b forming air-feeding paths are formed in at least a part of the inner surface, facing tubular portion 20, of cap 10. In contrast, in cap 10b according to the fourth embodiment, no groove is formed in the inner surface facing tubular portion 20b but grooves 11i, 11j are formed in tubular portion 20b instead. Specifically, tubular portion 20b has an outer surface facing cap 10b, and grooves 11i, 11j are formed in this outer surface.

In probe 25b, grooves 11i, 11j formed in tubular portion 20b form air-feeding paths allowing air to flow. Therefore, the air fed from blower 37 passes through the air-feeding paths formed in probe 25b to be supplied to the optical component of tubular portion 20b and mirror 1 mounted in cap 10b, for example. In three-dimensional scanner 100, air is supplied to the optical component of tubular portion 20b and mirror 1 mounted in cap 10b, for example, to thereby prevent fogging of the optical component of tubular portion 20b and mirror 1 mounted in cap 10b, for example.

As seen from the foregoing, tubular portion 20b according to the fourth embodiment has a surface facing cap 10b and grooves 11i, 11j forming air-feeding paths are formed in at least a part of this surface. Accordingly, with cap 10b mounted on tubular portion 20b, the air fed from blower 37 can be supplied to the optical component of tubular portion 20b and mirror 1 mounted in cap 10b, for example, through the air-feeding paths formed by grooves 11i, 11j and the inner surface of cap 10b.

The number of grooves 11i, 11j formed in tubular portion 20b is not limited to two but may be one or may be three or more. When a plurality of grooves are formed in tubular portion 20b, these grooves may differ from each other in terms of the size and/or the shape of the groove. Further, a groove formed in the cap may be combined with a groove formed in the tubular portion to thereby form an air-feeding path. Moreover, the configuration of grooves 11a, 11b, formed in cap 10 as described above is also applicable to grooves 11i, 11j formed in tubular portion 20b.

### Modifications

(a) The groove formed in the cap or the groove formed in the tubular portion in the first to fourth embodiments has an arc-shaped cross section facing the air-feeding direction. The cross-sectional shape, however, is not limited to this but may be another shape such as rectangular shape or wedge shape. Moreover, the air-feeding path formed between the cap and the tubular portion is not limited to the one formed in a part of the portion between the cap and the tubular portion, but may be formed along the whole boundary between the cap and the tubular portion.
(b) According to the above description of the cap in the first to fourth embodiments, the cap includes mirror 1. The cap, however, may not include an optical component such as mirror 1, as long as the cap is configured to direct light from a target to tubular portion 20.
(c) According to the above description of the first to fourth embodiments, the image capturing device is a three-dimensional scanner. The image capturing device, however, is not limited to this. For example, the image capturing device may be an image capturing device such as intraoral camera, optical coherence tomography (OCT) device, UV/IR/terahertz imaging device, or fluorescence imaging device, for example.

Although the present invention has been described and illustrated in detail, it is clearly understood that the same is by way of illustration and example only and is not to be taken by way of limitation, the scope of the present invention being interpreted by the terms of the appended claims.

## Claims

1. An image capturing device (100) for capturing an image of a target, the image capturing device comprising:
a tubular portion (20) including an optical component and configured to receive light from the target;
a housing (30) including an electronic component configured to process the light received from the tubular portion (20);
a cap (10) covering at least a part of the tubular portion (20); and
a blower (37) mounted in the housing (30) and configured to supply air to at least one of the cap (10) and the optical component included in the tubular portion (20), wherein
the cap (10) and the part of the tubular portion (20) covered by the cap (10) define at least one air-feeding path configured to pass the air supplied from the blower (37).

2. The image capturing device (100) according to claim 1, wherein a groove (11a, 11b; 11c, 11e; 11d, 11f; 11i, 11j) forming the air-feeding path is formed in at least a part of an inner surface, facing the tubular portion (20), of the cap (10).

3. The image capturing device (100) according to claim 1 or 2, wherein a groove (11a, 11b; 11c, 11e; 11d, 11f; 11i, 11j) forming the air-feeding path is formed in at least a part of a surface, facing the cap (10), of the tubular portion (20).

4. The image capturing device (100) according to any one of claims 1 to 3, wherein the air-feeding path has a substantially constant cross-sectional area facing an air-feeding direction in the air-feeding path.

5. The image capturing device (100) according to any one of claims 1 to 4, wherein the air-feeding path has a height measured from the tubular portion (20) to the cap (10) and the height decreases gradually in a direction from the housing (30) to the cap (10).

6. The image capturing device (100) according to any one of claims 1 to 5, wherein
the air-feeding path has a width perpendicular to a height, measured from the tubular portion (20) to the cap (10), of the air-feeding path, and
in the vicinity of the optical component included in the tubular portion (20), the width of the air-feeding path is substantially identical to or more than a width of the optical component included in the tubular portion.

7. The image capturing device (100) according to any one of claims 1 to 6, wherein the cap (10) includes an optical component configured to direct light from the target to the tubular portion (20).

8. The image capturing device (100) according to claim 7, wherein
the air-feeding path has a width perpendicular to a height, measured from the tubular portion (20) to the cap (10), of the air-feeding path, and
in the vicinity of the optical component included in the cap (10), the width of the air-feeding path is substantially identical to or more than a width of the optical component included in the cap.

9. The image capturing device (100) according to any one of claims 1 to 8, wherein the cap (10) includes a regulator configured to regulate the air supplied through the air-feeding path to cause the air to flow to at least one of the cap (10) and the optical component included in the tubular portion (20).

10. The image capturing device (100) according to any one of claims 1 to 9, wherein the cap (10) is mountable on and detachable from the tubular portion (20).

11. The image capturing device (100) according to any one of claims 1 to 10, wherein the at least one air-feeding path comprises a plurality of air-feeding paths defined between the cap (10) and the tubular portion (20).

12. The image capturing device (100) according to claim 7 or 8, wherein
the image capturing device (100) is configured to capture an image of an inside of an oral cavity, and
the optical component of the cap (10) is a mirror configured to reflect light from the target in the oral cavity toward the tubular portion (20).

13. A cap (10) mountable on and detachable from a tubular portion (20) of an image capturing device (100), the image capturing device comprising: the tubular portion (20) including an optical component and configured to receive light from a target; a housing (30) including an electronic component configured to process the light received from the tubular portion (20); and a blower (37) mounted in the housing (30) and configured to supply air,
the cap (10) comprising:
a reflector configured to direct the light from the target to the tubular portion (20); and
a cover member holding the reflector at one end while covering the tubular portion (20), wherein
a groove (11a, 11b; 11c, 11e; 11d, 11f; 11i, 11j) is formed in at least a part of an inner surface, facing the tubular portion (20), of the cover member, and
with the cap (10) mounted on the tubular portion (20), the groove (11a, 11b; 11c, 11e; 11d, 11f; 11i, 11j) forms an air-feeding path configured to supply the air from the blower (37) to at least one of the reflector and the optical component included in the tubular portion (20).
